# EUROPEAN PATENT SPECIFICATION

(11) **EP 2 343 342 B1**
(45) Date of publication and mention of the grant of the patent: **16.09.2015**
(21) Application number: 11163604.9
(22) Date of filing: 23.03.2007
(51) Int. Cl.: A61L 27/52, A61L 15/60, A61K 9/06, A61L 24/00, C08G 18/50, C08G 18/67, C08G 18/32, C08L 101/14, C08G 18/80

(54) **STRONG REVERSIBLE HYDROGELS**
STARKE UMKEHRBARE HYDROGELS
HYDROGELS FORTEMENT RÉVERSIBLES

(43) Date of publication of application: 13.07.2011
(62) Divisional of application: 07104756.7
(73) Proprietor: SupraPolix B.V., 5612 AX Eindhoven (NL)
(72) Inventor: Baughman, Trayvis Wayne, 5644 GG, Eindhoven (NL); Hoorne - van Gemert, Gaby Maria Leonarda, 6041 GH Roermond (NL); Janssen, Henricus Marie, 5625 AM, Eindhoven (NL); Meijer, Egbert Willem, 5583 GC, Waalre (NL); Bosman, Anton Willem, 5613 LK Eindhoven (NL)
(74) Representative: Nederlandsch Octrooibureau

(56) References cited:
- EP-A- 0 744 428
- WO-A-2004/052963
- WO-A-2006/118460
- WO-A-2006/118461
- WO-A1-2005/042641
- US-A- 5 631 337
- US-A1- 2004 161 394
- LANGE R F M ET AL: "HYDROGEN-BONDED SUPRAMOLECULAR POLYMER NETWORKS", JOURNAL OF POLYMER SCIENCE, POLYMER CHEMISTRY EDITION, INTERSCIENCE POUBLISHERS, NEW YORK, NY, US, vol. 37, 1999, pages 3657-3670, XP002907040, ISSN: 0360-6376
- YAMAUCHI K ET AL: "Thermoreversible poly(alkyl acrylates) consisting of self-complementary multiple hydrogen bonding", MACROMOLECULES, ACS, WASHINGTON, DC, US, vol. 36, no. 4, 2003, pages 1083-1088, XP002260387, ISSN: 0024-9297
- KY HIRSCHBERG J H K: "Ureidotriazine-based Supramolecular Copolymers", MACROMOLECULES, ACS, WASHINGTON, DC, US, vol. 36, no. 5, 2003, pages 1429-1432, XP002366465, ISSN: 0024-9297

## Description

### FIELD OF THE INVENTION

This invention relates to new hydrogel materials that comprise water gellants comprising hydrophilic polymers to which several hydrogen bonding units are covalently attached via an apolar motif so that they are cross-linked in a reversible supramolecular way by hydrogen bonds. As the water gellants are physically or non-covalently cross linked, the hydrogel materials are more easily processed. In addition, fine-tuning of the material properties of such hydrogel materials (e.g. mechanical strength or elasticity, degradation behavior) can be controlled more easily.

### BACKGROUND OF THE INVENTION

Structurally, hydrogels are three-dimensional networks of polymer chains with a high content of absorbed water molecules. Hydrogels find applications in for example medical applications including bone transplants and tissue adhesives, drug delivery systems, pharmaceuticals and in water management.

Hydrogels can occur in the cross-linked form or in the uncross-linked form. Cross-linking usually provides higher viscosities due to an apparent or real increase of the molecular weight and often results into the formation of gels.

Cross-linking can be achieved chemically by the formation of covalent bonds or physically by the formation of e.g. hydrogen bonds or ionic interactions. Obviously, cross-linking can also be achieved both chemically and physically.

Chemical cross-linking of hydrophilic polymers is a general and often applied route to obtain hydrogels. In order to be able to administer or process these gels, prepolymers are dissolved in water and are then polymerized resulting in (in situ) hydrogel formation. Hydrogellation procedures are often based on the use of acrylic or methacrylic macromonomers that are not preferred in (biomedical) applications, because of their inherent toxicity and because they usually require an auxiliary, potentially hazardous, initiator for polymerization. Moreover, chemically cross-linked hydrogels lack reversibility and are limited in their degradation behavior, as poly(acrylate)s and poly(methacrylate)s are not biodegradable. For example, US 5.410.016 discloses hydrogels based on copolymers of poly(ethylene glycol) with poly(DL-lactide) containing pendant acrylate functions that are cross-linked in situ. WO 01/44307 discloses hydrogels based on polyvinyl alcohol modified with pendant acrylate and methacrylate groups that are chemically cross-linked in situ. Hence, according to these prior art references, an irreversible cross-linked hydrogel is obtained by starting from water processable prepolymers that contain reactive groups.

Hydrogels based on natural polymers, especially collagen, are biocompatible and mostly thermally reversible (Mooney et al. Chem. Rev. 101, page 1869, 2001). However, the mechanical properties of these gels are limited and hardly, if at all, tunable. Especially the mechanical strength in these materials is too low, and often an additional chemical modification is required to make them stronger. However, this results in a reduced biocompatibility and a reduced biodegradation.

US 4.942.035 and US 5.548.035 disclose hydrogels based on block-copolymers in which hydrophilic blocks are alternated by hydrophobic blocks. For example, US 4.942.035 discloses a triblock copolymer consisting of a polyethylene glycol middle block surrounded by two poly(D,L-lactide-co-glycolide) polyester blocks (weight ratio of polyester to PEG at least 1) was prepared and showed gelling behaviour in water. The hydrogels are formed because of phase separation of the hard hydrophobic polyester block, and consequently the relative amount of the hydrophobic polymer needs to be high to counterbalance the hydrophilicity of the polyethylene glycol block to guarantee the gelling behaviour. Therefore, the range of mechanical properties of these gels is limited - for examples with respect to the elasticity - as these properties are mainly governed by the hard block.

WO 99/07343 discloses thermally reversible hydrogels intended for uses in drug delivery applications that are based on a hydrophilic polyethylene glycol block and hydrophobic PLLA (poly-L-lactic acid) blocks. The gelling is governed by the presence of the crystalline hard blocks formed by the PLLA. The presence of the crystalline PLLA-blocks limits the mechanical properties and the biodegradation of these materials to a great extent.

US 6.818.018 discloses hydrogels that can be formed in a mammal *in situ* by providing a system comprising a first polymer that is capable to form physical cross-links and a second polymer that is capable to form chemical cross-links. The first polymer may be selected from a wide group of materials including ionomers whereas the second polymer may be selected from virtually any material that has chemical groups capable of forming covalent bonds.

US 5.883.211 discloses a thermo-reversible hydrogel comprising a physically cross-linked copolymer based on poly(acrylamide) containing up to six different monomers with hydrogen bonding N-substituent groups. The relative content of these monomers with hydrogen bonding N-substituent groups in the copolymer needs to be higher than 50% to display thermo-reversible gelling behaviour.

In general, "supramolecular chemistry" is understood to be the chemistry of physical or non-covalent, oriented, multiple (at least two), co-operative interactions. For instance, a "supramolecular polymer" is an organic compound that has polymeric properties - for example with respect to its rheological behaviour - due to specific and strong secondary interactions between the different molecules. These physical or non-covalent supramolecular interactions contribute substantially to the properties of the resulting material.

Supramolecular polymers comprising (macro)molecules that bear hydrogen bonding units can have polymer properties in bulk and in solution, because of the hydrogen bridges between the molecules. Sijbesma et al. (see US 6.320.018 and Science, 278, 1601) have shown that in cases where a self-complementary quadruple hydrogen bonding unit (4H-unit) is used, the physical interactions between the molecules become so strong that materials with much improved properties can be prepared.

A poly(ethylene-propylene) oxide co-polymer (PEO-PPO-polymer) having three alcohol end groups was modified with 4H-units (cf. Lange et al. J. Polym. Sci. A, 1999, 3657 and WO 02/098377). The modified polymer was soluble in organic solvents such as chloroform and THF and it appeared that the viscosity of the polymer was significantly effected by the polarity of the solvent. For example, addition of water to a solution of the polymer resulted in a significant decrease of the viscosity due to breaking of the hydrogen bonds between polymer molecules and formation of hydrogen bonds between polymer molecules and water molecules. However, the viscosity was still much higher than that of a reference-solution of a low molecular weight compound bearing one 4H-unit. Due to the relatively high PPO-content (63%) of the PEO-PPO copolymer, this material will hardly, if at all, dissolve in water and the polymers have not been tested in water.

EP A 1392222 discloses *inter alia* a telechelic poly(ethylene-propylene) oxide co-polymer (PEO-PPO-polymer) having three alcohol end groups that is modified with 4H-units resulting in a non-waterprocessable polymer. Nevertheless, in example C of this patent a hairstyling gel is disclosed with only 0.2 wt% of the PEO-PPO-polymer containing 4H-units, in an aqueous composition that further contains 1.0 wt% of a gelling agent based on cross-linked high molecular weight polyacrylate and 17 wt% ethanol as co-solvent. Apparently, the high ethanol content is needed to make the PEO-PPO polymer processable, and the polyacrylate is required to get a gel composition.

US 2003/0079644 discloses ink additives comprising 2 - 4 4H-units that are prepared from e.g. PEO-PPO polymers commercially available under the trade name VORANOL® from Dow Chemical Co., Midland, Michigan, US, and 2(6-isocyanato-hexylaminocarbonylamino)-6-methyl-4(1H)-pyrimidone. According to the Examples XIII - XVI of US 2003/0079644, the ink compositions may comprise up to 5 wt.% of the polymer and water in the range of about 18 to about 35 wt.% and these compositions would have a viscosity at about 25°C of no more than about 10 cP (cf. paragraphs [0174] and [0179] of US 2003/0079644). This implies that these polymers are not water gellants.

Kautz et al. (Macromolecules , 2006, 39, 4265) show by AFM measurements that in telechelic poly(ethylene-butylene) polymers modified with 4H-units, the phase separation of the 4H-units is more pronounced when polymer backbone and 4H-unit are connected via a urea group when compared to a urethane connecting group. The mechanical properties of the urea-based polymer are not better, but worse, as evidenced by tensile testing results. Also, the presented materials cannot be used for hydrogel formulations due to their lack of hydrophilicity.

WO 2006/118460 discloses hydrogel materials comprising water gellants that are comprised by hydrophilic polymers to which at least two 4H-units are covalently attached via urethane-alkyl moieties. However, it appeared that these hydrogel materials are insufficient in strength for several applications. In addition, in some examples a toxic Sn-catalyst is employed and the presence of residues of such catalysts are undesired, in particular when the hydrogel materials are intended for biomedical applications.

US 5.631.337 discloses a non-ionic, linear copolymer capable of forming a hydrogel, wherein the copolymer comprises a polyacrylamide backbone.

K. Yamauchi et al., Macromolecules 36, 1083, 2003**,** discloses thermo-reversible poly(alkyl acrylates) comprising 4H-units.

Because of the reviewed shortcomings of state-of-the-art hydrogels, there is a need for synthetic polymers that are able to gel water reversibly, implying that the hydrogels can be switched between a gelled state and a liquid state. This would facilitate easy processing and administration of these hydrogels. In addition, it is desired that reversible hydrogels can be made that are elastic and that have high strengths. It would also be advantageous to be able to make biodegradable reversible hydrogels. Finally, in view of biomedical applications for hydrogels, it would be very beneficial to be able to make water gellants and their hydrogels in a quality-controlled fashion.

### SUMMARY OF THE INVENTION

It is an object of the present invention to provide new hydrogels comprising a polymeric water gellant, water and optionally another solvent and a process to prepare such new hydrogels, preferably without the use of a toxic metal-based catalyst. With this invention, easy preparation of hydrogels is enabled without the need for chemical cross-linking procedures. The hydrogels according to the invention combine easy processing and administration with good and tunable mechanical properties, while it is optional to make the hydrogels biodegradable.

The present invention therefore relates to a hydrogel comprising:
(a) 0.3 - 50.0 wt.%, based on the total weight of the hydrogel, of a water gellant wherein the water gellant is obtainable by converting a hydrophilic backbone P, wherein P is selected from the group consisiting of polyethers, polyesters, and copolymers of such polymers, in a first step into an amine functional prepolymer according to the formula P-[L]ₙ, wherein in a second step a simple hydrogen bonding unit is introduced at the termini of the hydrophobic linker L by reacting the amine functional prepolymer P-[L]ₙ with a mono- or difunctional compound selected from the group consisting of (di)isocyanates, (di)thioisocyanates, (di)amines which are optionally activated, (di)acid chlorides, (di)esters which are optionally activated, (di)ols which are optionally activated, (di)acryl amides, (di)methacryl amides, and (di)thiols which are optionally activated, wherein P and L are connected to each other by moieties selected from the group consisting of (thio)urea, (thio)urethane, amide, ester, carbonate, secondary amine, tertiary amine and ether moieties,
   wherein:
   n is in the range of 1.8 to 10;
   L is a hydrophobic linker selected from the group consisting of cyclic, linear or branched C₂ - C₂₄ alkylene groups, C₆ - C₂₄ arylene groups, C₇ - C₂₄ alkarylene groups and C₇ - C₂₄ arylalkylene groups, wherein the alkylene groups, arylene groups, alkarylene groups and arylalkylene groups optionally, but not preferably, comprise 1 - 5 heteroatoms selected from the group consisting of O, N and S; and
(b) 50.0 to 99.7 wt.% water.

In this invention, the water gellant is constructed from a polymer backbone P to which at least 1.8 urea or amide hydrogen bonding units are connected via a hydrophobic linker L. The urea or amide functions are more simple hydrogen bonding units than the 4H-unit.

### DETAILED DESCRIPTION OF THE INVENTION

It was surprisingly found that hydrogels based on water gellants comprising hydrophilic polymers comprising simple hydrogen bonding units could be improved in their mechanical performance when the simple hydrogen bonding unit was linked to the polymer backbone with a hydrophobic linker L, where this hydrophobic linker L was covalently connected to the simple hydrogen bonding unit with an urea or an amide moiety. The novel hydrophilic polymers are prepared by a versatile synthetic method. Moreover, this method does not require toxic (metal) catalysts, which makes the water gellants more suitable for biomedical applications.

The reversibility of the supramolecular hydrogen bonding interaction allows for reversible switching between a gelled state and a liquid state by changing the temperature, the concentration of polymeric gellant, or the polarity or ionic strength of the solvent, and therefore makes it possible to easily process or administer the hydrogel for the desired application. Moreover, the reversible supramolecular interaction can also favour the biodegradation of the hydrogel material.

In this document, the structure of the water gellant according to this invention and its preparation are disclosed, as well as the components of the gellant: (i) the simple hydrogen bonding unit, (ii) the polymer backbone P and (iii) the hydrophobic linker L. Finally, the hydrogels that can be prepared from the newly prepared water gellants are disclosed.

### GENERAL DEFINITIONS

An urea moiety as indicated in this document is to be understood as a moiety according to the formula:

-NRₐ-C(X)-NRₐ-

wherein X is O or S, preferably O, Rₐ is, independently, a hydrogen atom or a linear, branched or cyclic C₁ - C₁₂ alkyl group, preferably hydrogen, or a linear, branched or cyclic C₁ - C₆ alkyl group, most preferably a hydrogen.

An amide moiety as indicated in this document is to be understood as a moiety according to the formula:

-NRₐ-C(X)-

wherein X and Rₐ are as described above.

An urethane moiety as indicated in this document is to be understood as a moiety according to the formula:

-NRₐ-C(X)-X-

wherein X and Rₐ are as described above (X can independently be O or S).

An ester moiety as indicated in this document is to be understood as a moiety according to the formula:

-C(X)-X-

wherein X is as described above (X can independently be O or S).

A carbonate moiety as indicated in this document is to be understood as a moiety according to the formula:

-X-C(X)-X-

wherein X is as described above (X can independently be O or S).

An amine moiety as indicated in this document is to be understood as a moiety according to the formula:

-N(Rₐ)-

wherein Rₐ is as described above.

An ether moiety as indicated in this document is to be understood as a moiety according to the formula:

-X-

wherein X is as described above.

An isocyanate group is to be understood as a -NCX group, wherein X is as described above.

Acrylate and methacrylate groups are to be understood as R_{b}-C(=CH₂)-C(X)-X- groups, wherein X is as described above (X can independently be O or S), and wherein R_{b} is hydrogen or methyl. It is, however, preferred that in acrylate and methacrylate groups X is O.

Acrylamide and methacrylamide groups are R_{b}-C(=CH₂)-C(X)-N(Rₐ)- groups, wherein X and Rₐ are as described above, and wherein R_{b} is hydrogen or methyl. It is, however, preferred that in acrylamide and methacrylamide groups X is O.

The verb "to comprise" as is used in this description and in the claims and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there is one and only one of the elements. The indefinite article "a" or "an" thus usually means "at least one".

### THE STRUCTURE OF THE WATER GELLANT AND ITS PREPARATION

The water gellant of this invention has the general structure

(P)-(L)-(SHBU),

where P represents the polymer backbone to which the simple hydrogen bonding unit (SHBU) is covalently connected via the hydrophobic linker L.

The water gellant has a molecular weight of 1200 to 1.000.000, preferably 2000 to 100.000, more preferably 3000 to 80.000, more preferably 5000 to 50.000 and most preferably 7.500 to 21.000 Dalton.

The simple hydrogen bonding units can be attached to the polymer backbone P via the hydrophobic linker L in any way, e.g. by grafting onto the polymer backbone, by attachment to multiple - i.e. two or more - end groups of the polymer backbone, or the simple hydrogen bonding units can be an integral part of the backbone of the polymer that constitutes the water gellant. As will be understood by the person skilled in the art, the simple hydrogen bonding units may also be attached by a combination of these bonding modes.

It is preferred that the polymer backbone P and the hydrophobic linker L are connected via a (thio)urea, (thio)urethane, amide, ester, carbonate, secondary amine, tertiary amine or ether moiety, more preferably a urethane, urea or ether group, most preferably a urethane group.

The hydrophobic linker L is preferably connected to the simple hydrogen bonding unit via a (thio)urea, (thio)urethane, amide, ester, carbonate, secondary amine, tertiary amine or ether moiety. It is, however, more preferred that the hydrophobic linker L and the simple hydrogen bonding unit are connected via a (thio)urea or an amide moiety, even more preferably via a urea or amide moiety, most preferably via a urea moiety.

More preferably, the water gellant of this invention has the structure P-[L-(4H)]ₙ or the structure [P-L-(SHBU)-L-]ₚ. Most preferably, the water gellant has the general structure [P-L-(SHBU)-L-]ₚ.

In the structure P-[L-(SHBU)]ₙ, structure (A), the polymer backbone P is multifunctional and has n simple hydrogen bonding units connected to it via the hydrophobic linker L. The functionality n is at least 1.8, and is preferably 1.8 to 10, more preferably 1.8 to 5, even more preferably 1.8 to 3, most preferably 1.8 to 2, in which case the water gellant is preferably telechelic and has the general structure:

(SHBU)-L-P-L-(SHBU),

in which the backbone P is at each of the two sides connected to a simple hydrogen bonding unit via the hydrophobic linker L. Due to a somewhat lesser definition of the telechelic material, the functionality n may be as low as 1.8, but is preferably higher than 1.9, most preferably higher than 1.95.

In the structure [P-L-(SHBU)-L-]ₚ, structure (B), the polymer backbone P is covalently chain extended with the SHBU-unit via the hydrophobic linker L. Here, the number of repeating units p is at least 2, more preferably at least 3, most preferably at least 4. It is preferred that p is not higher than 25.

The hydrophobic linker L can be introduced into the water gellant by the method: the hydrophobic linker L is covalently attached to the polymer backbone P and thereafter the resulting functional prepolymer is connected to a simple hydrogen bonding unit building block to produce the water gellant.

In this method the functional prepolymer is denoted as P-[L]ₙ, where n represents the number of hydrophobic groups that are attached to the polymer backbone P. Preferably, n is at least 1.8, more preferably n is 1.8 to 10, more preferably 1.8 to 5, even more preferably 1.8 to 3, most preferably 1.8 to 2, in which case the prepolymer is preferably telechelic and can be denoted as L-P-L, where the backbone P is at each of the two sides connected to a functional hydrophobic group L. Due to a somewhat lesser definition of the telechelic material, the functionality n may be as low as 1.8, but is preferably higher than 1.9, most preferably higher than 1.95. The functional groups attached to the hydrophobic linkers L are preferably secondary or primary amine groups, most preferably primary amine groups.

Accordingly, the present invention relates to a method for the preparation of a water gellant having the formula:

P-[L-(SHBU)]ₙ (A)

or

[P-L-(SHBU)-L-]ₚ (B)

wherein in a first step a polymer backbone P is converted into a prepolymer according to the formula P-[L]ₙ, and wherein in a second step the simple hydrogen bonding unit is introduced at the termini of the hydrophobic linker moiety L, wherein P, L and the simple hydrogen bonding unit are connected to each other by moieties selected from the group consisting of (thio)urea, (thio)urethane, amide, ester, carbonate, secondary amine, tertiary amine and ether moieties. As will be apparent to the skilled person, the polymer backbone P, the hydrophobic linker L and the simple hydrogen bonding unit are furnished with functional groups that are capable in providing the connecting moieties described above.

Accordingly, the present invention also relates to a method for the preparation of a water gellant having the formula shown above. The backbone P, the linker L and the simple hydrogen bonding unit are connected to each other by moieties selected from the group consisting of (thio)urea, (thio)urethane, amide, ester, carbonate, secondary amine, tertiary amine and ether moieties. As will be apparent to the skilled person, the polymer backbone P, the hydrophobic linker L and the simple hydrogen bonding unit are furnished with functional groups that are capable in providing the connecting moieties described above.

In this invention, the amine functional prepolymer P-[L]ₙ as prepared in above mentioned method is reacted with a mono- or difunctional compound to produce water gellants in which the polymer backbone P is linked to a simple hydrogen bonding unit via the hydrophobic linker L. The simple hydrogen bonding unit can be any hydrogen bonding unit known in the art, but is preferably a (thio)urea, an amide, or a (thio)urethane. More preferably, it is an urea or an amide, most preferably an urea. Accordingly, the mono- or difunctional compounds are preferably (di)isocyanates, (di)thioisocyanates, (di) amines which are optionally activated, (di)acid chlorides, (di)esters which are optionally activated, (di)ols which are optionally activated, (di)acryl amides, (di)methacryl amides or (di)thiols which are optionally activated, preferably (di)isocyanates, (di) activated amines or (di) activated esters, most preferably (di)isocyanates. Non-limiting examples are hexylisocyanate, dodecylisocyanate, 1,6-hexyldiisocyanate, 1,10-decyldiisocyanate, isophorone diisocyanate, adipoyl chloride, glutaryl chloride, succinyl chloride, undecenoyl chloride, and the like.

Also in this embodiment, for P-[L]ₙ, n is preferably 1.8 to 10, more preferably 1.8 to 5, even more preferably 1.8 to 3, most preferably 1.8 to 2, in which case the amine functional polymer is preferably telechelic, and n is preferably 1.9 to 2, most preferably 1.95 to 2. When n = 1.8 to 2, the compounds with which P-[L]ₙ are reacted can be monofunctional (i.e. a capping reaction) or difunctional (i.e. a chain extension reaction), preferably difunctional. When n is greater than 2, the compound is monofunctional (i.e. a capping reaction).

The water gellant according to this embodiment of the invention has a molecular weight of 1200 to 1.000.000, preferably 2000 to 100.000, more preferably 3000 to 80.000, more preferably 5000 to 50.000 and most preferably 7.500 to 21.000 Dalton.

### THE SIMPLE HYDROGEN BONDING UNIT

The functional groups can be any functionalities known in the art, but are preferably (thio)isocyanates, (activated) amines, alcohols, thiols, (activated) esters, acrylates, methacrylates, acryl amides, methacryl amides or other vinyl groups, more preferably isocyanates, (activated) amines, acrylates, methacrylates or alcohols, even more preferably isocyanates or (activated) amines, most preferably isocyanates.

### THE POLYMER BACKBONE P

The polymer backbone P has a molecular weight of 250 to 200.000, preferably 1000 to 100.000, more preferably 2000 to 80.000, more preferably 3000 to 50.000 and most preferably 5000 to 30.000 Dalton.

The polymer backbone P is preferably a hydrophilic polymer, and according to this invention, a hydrophilic polymer is defined as a polymer having a solubility in water of at least 1 g/L.

P may represent any type of polymer backbone known in the art, such as preferably polyethers, polyesters, polyamides, polyacrylates, polymethacrylates, polyolefins, hydrogenated polyolefins, polysiloxanes, polycarbonates, (per)fluorinated polyethers, polyvinylenes, or co-polymers of such polymers. More preferably, the polymer backbone is a polyether, polyester, polyacrylate, polymethacrylate, polyolefin, hydrogenated polyolefin, polycarbonate, polyvinylene, or a co-polymer of such polymers. Even more preferred are polyethers, polyesters, or copolymers thereof. Most preferably, P is a polyether, preferably a polyglycol, preferably a polyethylene glycol or a poly ethylene-co-propylene glycol (random or block), most preferably a polyethylene glycol.

Although some of the above listed polymer backbones P themselves may not be hydrophilic *per se,* co-polymerizing them with the right amount of water soluble polymer, or use of a combination of these polymer backbones P can lead to a water gellant, as will be obvious to a person skilled in the art.

The polymers P that are used to prepare the water gellant of this invention can have different functional groups, such as for example alcohols, amines, thiols, isocyanates, carboxylic acids or combinations of these end groups. Preferably, the functional groups are alcohols or amines, most preferably alcohols, preferably primary alcohols. In this preferred case, the polymer backbone P is derived from a hydroxy functional polymer P-(OH)ₙ.

The functional polymer P that is used to prepare the water gellant of this invention can have different numbers (n) of functional groups. The average functionality n of the functional polymer is preferably 1.8 to 10, more preferably 1.8 to 5, even more preferably 1.8 to 3, and most preferably 1.8 to 2.

The polymers P that are used to prepare the water gellant of this invention can be combinations of polymers with different backbone compositions, different architectures, different molecular weights and/or a different (numbers of) functional groups. For example, one can use a telechelic polyethylene glycol with a molecular weight of 5000 Dalton in combination with a telechelic polyester with a molecular weight of 500 Dalton.

According to the first embodiment of this invention, the functional polymer P that is used to prepare the water gellant is a telechelic polymer, and this telechelic polymer is preferably hydroxy functional. One can represent this telechelic polymer as P-(OH)ₙ, with n (about) 2, or 1.8 to 2, more preferably 1.9 to 2, most preferably 1.95 to 2. The polymer P-(OH)₂ is preferably selected from the group consisting of polyethers, polyesters, polyamides, polycarbonates, polysiloxanes, hydrogenated polyolefins, polyortho esters, or lower molecular weight materials that are derived from dimerized fatty acids, such as Pripol and Priplast, both marketed by Uniqema BV, the Netherlands, more preferably, the prepolymer backbone P is selected from the group consisting of polyethers or polyesters, most preferably P is selected from polyethers, most preferably polyethylene glycols.

Examples of telechelic functional polymers P-(OH)₂ are (a) polyetherdiols having a polyoxyalkylene structure, such as polyethylene glycols, polypropylene glycols, poly(ethylene-co-propylene) glycols (random or block), poly(ethylene-block-propylene-block-ethylene) glycols (also known as Pluronics), polytetramethylene glycols (i.e. poly-tetrahydrofurans) or poly(ethylene-co-tetramethylene) glycols. Other examples are (b) polyesterdiols or copolyester diols, made by polycondensation of dicarboxylic acids and diols, or by polycondensation of hydroxyacids, or by ringopening polymerisation of e.g. ε-caprolactone, glycolide, lactide, δ-valerotactone, 1,4-dioxane-2-one, 1,5-dioxepan-2-one, oxepan-2,7-dione, and the like. Specific examples are poly ε-caprolactonediols, hydroxy terminated polyadipates or polyglutarates, such as hydroxy terminated poly(1,4-butylene adipate)s, hydroxy terminated poly(1,2-ethylene adipate)s, hydroxy terminated poly(1,4-butylene glutarate)s, hydroxy terminated poly(2-methyl-1,3-propylene adipate)s, hydroxy terminated poly(2-methyl-1,3-propylene glutarate)s, hydroxy terminated poly(2-methyl-1,5-pentylene adipate)s, polyesterdiols of polylactides, polyglycolides, poly(lactide-co-glycolide)s, poly(hydroxy butyrate)s, polyterephthalates such as polyethyleneterephthalates and polybutyleneterephthalates, polyisophthalates (e.g. hydroxy terminated copolymers of 5-NaSO₃-isophtatic acid, isophthalic acid, diethyleneglycol and bis-hydroxymethylene-cyclohexane, hydroxy terminated copolymers of isophtalic acid and 1,4-butanediol, hydroxy terminated copolymers of 5-NaSO₃-isophthalic acid, adipic acid, phthalic acid and 1,6-hexanediol) and polyphthalates such as poly(1,6-hexylene phthalate)s or hydroxy terminated copolymers of phthalic acid and diethyleneglycol. Further examples include (c) polyolefine diols or hydrogenated polyolefine diols, such as hydroxyl functionalized polybutadienes or hydroxyl functionalized hydrogenated poly(ethylene-butylene)s such as Kraton L-2203 or Nisso-type materials. Other examples are (d) hydroxy functionalized polycarbonates and co-polycarbonates based on glycols or made by ringopening polymerization of e.g. trimethylenecarbonate, 1,3-dioxepane-2-one, 1,3-dioxanone-2-on and 1,3,8,10-tetraoxacyclotetradecane-2,9-dione. Examples are hydroxy terminated poly(1,3-propanediol carbonate)glycols, poly(trirnethylenecarbonate)s, poly(1,6-hexanediol carbonate)glycols. More examples include (e) low molecular weight diols based on dimerized fatty acids such as Pripols and Priplasts, such as Pripol 2033, Priplast 3190 or Priplast 3192 (marketed by Uniqema BV, the Netherlands), (f) polysiloxanes such as α,ω- bis(6-hydroxy hexyl) polydimethylsiloxanes, α,ω-bis(oligo-ethyleneoxide) polydimethylsiloxanes, and (g) polyamides such as α,ω-dihydroxy-polyamides. Of course, examples are also (h) alcohol terminated co-polymers of the examples mentioned above, such as (block)-co-polymers of poly-caprolactone and ethylene glycol or (block)-co-polymers of poly-caprolactone and tetramethylene glycol. Preferred examples of hydroxy functional telechelics P-(OH)₂ are those of categories (a) and (b), more preferred are those of (a). Within category (a), the polymer P-(OH)₂ is preferably selected from the group consisting of polyethylene glycol, poly(tetrahydrofuran) or poly(ethylene-co-propylene) glycol (random or block). Most preferably, the polymer within category (a) is polyethylene glycol.

As an alternative, although less preferred, telechelic polyoxyalkylene amines may be used, such as polyethylene glycols or poly(ethylene-co-propylene) glycols with terminal amino groups. Examples are Jeffamines® as sold by Huntsman.

According to the second embodiment of this invention, the functional polymer P that is used to prepare the water gellant is selected from the group consisting of multifunctional polymers, functional star polymers, functional grafted polymers, functional (hyper)branched polymers and functional dendritic polymers. More preferably, in this embodiment, the functional polymer is a functional star polymer, a functional grafted polymer or a functional branched polymer, most preferably a functional star polymer. Also in this embodiment, the functional polymers are preferably hydroxy functional, and can then be represented by P-(OH)ₙ with n > 2.

Functional star polymers P-(OH)ₙ are preferably polyethers or polyesters, more preferably polyethers. Polyester star polymers are preferably preparable by ring opening polymerization of suitable monomers such as lactones (e.g. ε-caprolactone, lactides, glycolides, and the like), using a multifunctional alcohol initiator, such as glycerol, erythritol, 1,1,1-tris(hydroxymethyl)ethane, 1,1,1-tris(hydroxymethyl)-propane, pentaerythritol, saccharoses, and derivatives thereof A specific polyester star polymer is a polycaprolactone triol prepared from a 1,1,1-tris(hydroxymethyl)propane core. Polyether star polymers are preferably obtainable by polymerizing ethylene oxide, propylene oxide or a mixture thereof, using a multifunctional initiator as core molecule. Poly(ethylene glycol) star polymers having OH functional groups can for example be prepared by living anionic polymerization using divinyl benzene as the core molecule, whereafter the polyethylene oxide arms are grown outwards from the core. Other methods include the use of dendritic core molecules having a multitude of reactive groups from which polyethylene oxide arms can grow in outward direction. More simple initiators, e.g. glycerol, erythritol, 1,1,1-tris(hydroxyrnethyl)ethane, 1,1,1-tris(hydroxymethyl)propane, pentaerythritol, saccharoses, and derivatives thereof, can be used as well as core molecule. Most preferably, the functional star polymer is a poly(ethylene oxide) polymer having OH end-groups according to Formula (1):

Z-[(CH₂CH₂O)ᵣ-OH]ₛ Formula (1)

wherein Z represents the multifunctional core, r is in the range of 4 to 250 and s is in the range of 3 to 10, more preferably s is in the range of 3 to 5, and most preferably s is (about) 3.

Examples of functional hyperbranched polymers having OH-groups are Hybrane® from DSM, The Netherlands, and Bottorn® from Perstorp, Sweden. Examples of branched polymers are branched polyglycerols or branched polyesters having OH-groups. Branched polyglycerols having OH end-groups can for example be obtained by Lewis acid catalyzed polymerization of glycidol.Branched polyesters can for example be prepared by polymerizing a dicarboxylic acid and/or a dicarboxylic anhydride, a diol such as polyethylene glycol and a multifunctional alcohol having at least three OH groups, e.g. trimethylol propane or glycerol. A specific example of a branched polyester is a trifunctional poly(2-methyl-1,3-propylene)adipate.

Functional grafted polymers P-(OH)ₙ are obtainable by (co)polymerization of vinyl monomers. Preferably, these vinyl monomers are selected from the group consisting of:
(a) acrylate or methacrylate monomers according to Formula (2), 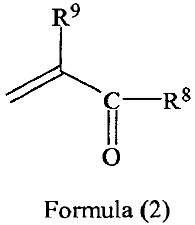 wherein R⁸ is independently selected from the group consisting of:
   (i) OH;
   (ii) C₁ - C₁₂ linear or branched alkoxy, optionally substituted with 1 - 6 hydroxy groups;
   (iii) amide according to the formula -N(R¹⁰)₂ wherein R¹⁰ can be hydrogen or C₁ - C₆ linear or branched alkyl, optionally substituted with 1 - 6 hydroxy groups;
   (iv) ammonium salt according to the formula -[N(R¹⁰)₃]⁺X⁻, wherein R¹⁰ is as defined for (iii) and X is a halogen atom; and
   (v) a group according to the Formula (3) 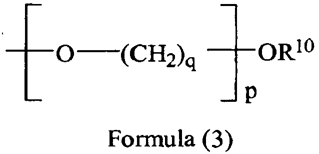 wherein R¹⁰ is as defined for (iii) and p is 1 - 50 and q = 2 or 3 and where (CH₂)_{q} can be linear or branched; and wherein R⁹ is hydrogen or methyl;
(b) C₁ - C₁₂ linear or branched alkyl vinyl ether;
(c) vinyl alcohol;
(d) C₂ - C₁₂ α-alkenylene ω-sulfonate having an alkaline earth metal cation or an alkali metal cation;
(e) C₇ - C₁₂ vinylaryl sulfonate according to Formula (4) 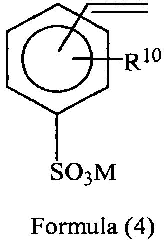 wherein R¹⁰ is as defined for (iii) and M is an alkaline earth metal or an alkali metal cation;
(f) CH₂=CH-R¹¹, wherein R¹¹ is selected from the group consisting of pyrrolyl, imidazolyl, pyrazolyl, pyridinyl, pyrazinyl, pyrimidinyl, pyrrolidyl, indolyl, indolizinyl, indazolyl, purinyl, quinolizinyl, quinolinyl, isoquinolinyl, phthalizinyl, naphtypyridinyl, quinoxalinyl, quinazolinyl, cinnolinyl, pteridinyl, pyrrolidonyl, pyrrolinyl, pyrrolidinyl, imidazolidinyl, imidazolinyl, pyrazolidinyl, pyrazolinyl, piperidyl, piperazinyl, indolinyl, isoindolinyl, morpholinyl, isoxazolyl, furazinyl, and isothiazolyl;
(g) CH2=CH-O-C(O)R¹², wherein R¹² is selected from the group consisting of C₁ - C₆ linear or branched alkyl;
(h) CH₂=CH-CH₂OR¹³, wherein R¹³ is selected from the group consisting of hydrogen and C₁ - C₆ linear or branched alkyl; and
(i) N-vinyl lactams according to the Formula (5) 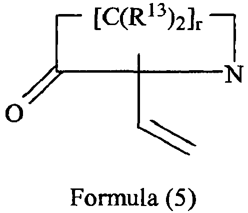 wherein R¹³ is as defined for (h) and r is 2 - 6.

More preferably, grafted polymers P-(OH)ₙ are obtainable by co-polymerizations of monomers selected from groups (a) and (c), optionally co-polymerised with monomers of groups (b), (d) - (i). Most preferably, the monomers are selected from group (a), where preferably hydroxyethyl methacryalate (HEMA) or hydroxyethyl acrylate (HEA) are used as co-monomer.

Examples of functional dendritic polymers are poly(propylene imine) dendrimers, poly(amido amino) dendrimers (both these have amine end groups), or arborol type dendrimers that are alcohol terminated dendrimers.

The multifunctional polymer P according to the second embodiment of the invention can be of natural origin or of synthetic origin. However, according to the invention, it is preferred that the polymer is of synthetic origin, and many examples are given above. If the polymer is of natural origin, it is preferred that it is selected from the group consisting of proteins, e.g. proteins selected from the group consisting of collagen, gelatine, or fibrin, and polysaccharides, e.g. polysaccharides selected from the group consisting of hyaluronate, agar, agarose, xantham gums, natural gum, alginate, chitosan or inulin, or synthetic derivatives from them, preferably collagen or chitosan.

### THE HYDROPHOBIC LINKER L AND ITS INTRODUCTION INTO THE WATER GELLANT

### The hydrophobic linker L

The hydrophobic linker L between the polymer backbone P and the simple hydrogen bonding unit is selected from the group consisting of cyclic, linear or branched C₂ - C₂₄ alkylene groups, C₆ - C₂₄ arylene groups, C₇ - C₂₄ alkarylene groups and C₇ - C₂₄ arylalkylene groups, wherein the alkylene groups, arylene groups, alkarylene groups and arylalkylene groups optionally, but not preferably, comprise 1 - 5 heteroatoms selected from the group consisting of oxygen, nitrogen and sulphur.

More preferably, the hydrophobic linker L is selected from the group consisting of cyclic, linear or branched C₄ - C₂₀ alkylene groups and C₆ - C₂₀ arylene groups, wherein the alkylene groups or arylene groups optionally, but not preferably, comprise 1 - 3 heteroatoms, preferably oxygen. Even more preferably, L is selected from the group consisting of cyclic, linear or branched C₆ - C₂₀ alkylene groups. Most preferably, L is selected from cyclic, linear or branched C₆ - C₁₂ alkylene groups.

The linker L may also be perfluorinated or partly fluorinated versions of the linkers described above, but this is not preferred.

### Introduction of the linker L into the water gellant of this invention

The hydrophobic linker L can be introduced into the water gellant by the following method: the linker L is covalently attached to the polymer backbone P and the resulting functional prepolymer is connected to a simple hydrogen bonding unit building block to produce the water gellant. This method is described here in detail.

The hydrophobic linker L can be covalently connected to the polymer backbone P using any reaction or sequence of reactions known in the art. Type of reactions may include coupling reactions such as for example etherifications, esterifcations, amidation reactions, carbamate forming reactions or Michael-type additions, in which cases P and L are connected via ether, ester, amide, urethane or amine moieties, respectively. Other type of reactions that may be involved in the introduction of the linker L are protection or deprotection reactions and activation reactions, as will be appreciated by persons skilled in the art.

In this synthetic method, functional polymers P - that are preferably hydroxy functional polymers P-(OH)ₙ - are converted in a two step procedure to give prepolymers with functional hydrophobic linker groups L attached to them. The first step involves the activation of a functional polymer with a (thio)carbonyl-containing reactant to acquire an activated polymer, and the second step involves reaction of the activated polymer with (an excess of) a difunctional compound containing the hydrophobic linker L. The difunctional compound can be any difunctional compound having at least one nucleophilic group, such as a diamine, a diol, an amino-alcohol, a dithiol, an amino acid or the like. Preferably, the difunctional compound is a diamine, so that amine functional prepolymers are prepared.

For the preferred case where the starting polymer P is hydroxy functional and where the difunctional compound is a diamine, the two reaction steps are given by Reactions (i) and (ii): 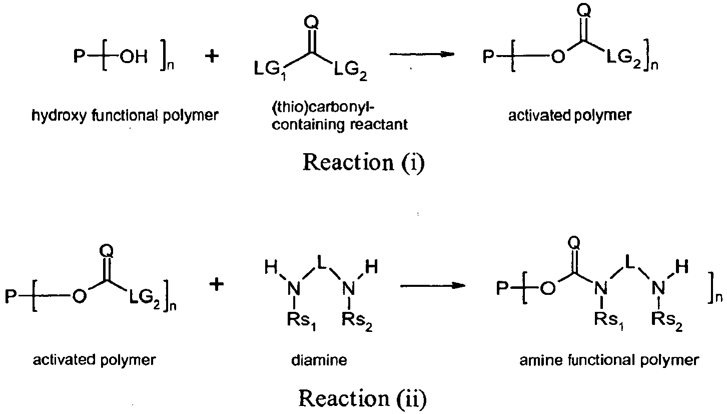 wherein P is the polymer backbone, L is the hydrophobic linker, and n is the (average) functionality of the hydroxy functional polymer and all three are defined as above, where Q is an oxygen or a sulphur atom, preferably an oxygen atom, where Rs₁ and Rs₂ are residues that are related to the used diamine and may represent independently any residue known in the art, such as hydrogen or C₁-C₆ alkyl groups, so that Rs₁ and Rs₂ may provide extra hydrophobicity to the spacer between the polymer backbone P and the simple hydrogen bonding unit, and where LG₁ and LG₂ both independently represent a leaving group, and they may represent any leaving group known in the art, such as for example imidazoles, triazoles, halogens, N-hydroxysuccinimides, (substituted) phenols and carboxylates. LG₁ and LG₂ may be the same leaving group, for example, LG₁ and LG₂ may both be an imidazole group, so that in case of Q being an oxygen, the carbonyl-containing reactant is 1,1'-carbonyldiimidazole (CDI).

In the preferred embodiment of this invention where n is (about) 2, and where the functional polymer P-(OH)₂ is telechelic, Reactions (i) and (ii) translate into Reactions (iii) and (iv), that are given below, with P, Q, L, Rs₁, Rs₂, LG₁ and LG₂ being defined as above.

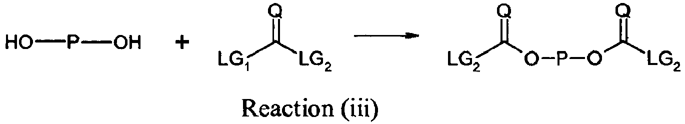

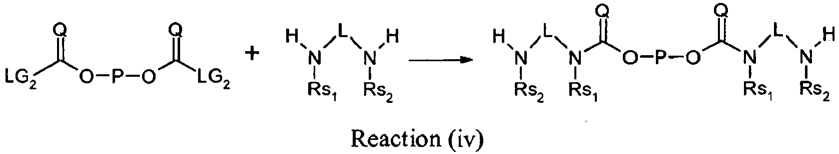

The applied synthetic methodology that is highlighted in Reactions (i) to (iv) is simple (e.g. no use of protective group chemistry), is versatile (i.e. able to produce many types of amine end groups with incorporated linkers L on a broad range of polymer backbones P), does not require metal catalysts for coupling reactions, circumvents the use of isocyanate chemistry and/or the use of metal hydrogenation catalysts that are routinely applied in the synthesis of amine functional polymers, can produce stable multifunctional polymeric materials with a high degree of definition (i.e. the amine functionality is high), and allows for upscaling using quality control conditions. Therefore, according to a very preferred embodiment of the present invention, the water gellant is prepared using the described synthetic method wherein in a first step a telechelic functional polymer P-(OH)₂ is reacted with a carbonyl compound of the formula LG₁-C(Q)-LG₂, wherein LG₁, LG₂ and Q are as defined above, to form a functionalised prepolymer intermediate, and wherein in a second step the functionalised prepolymer intermediate is reacted with a diamine H(R_{S1})N-L-N(Rs₂)H.

### Specifications to the reactions (i) to (iv)

LG₁ and LG₂ in the (thio)carbonyl containing reactant that is used in Reactions (i) and (iii) both represent a leaving group. Leaving groups LG₁ and LG₂ can be any leaving group known in the art, and can for example and preferably be imidazole or a derivative thereof, triazole or a derivative thereof, a halogen, N-hydroxysuccinimide or a derivative thereof, a (substituted) phenol, a (substituted) thiophenol, a thiol, certain alcohols (e.g. 1,1,1-trichloro methanol, 2-halo, 2,2-dihalo alcohols, 2,2,2-trihalo or 2-methylsulfonyl ethanol, or (substituted) benzyl alcohols), a carboxyl derivative, an amide (e.g. ε-caprolactam), a tosylate, a mesylate and a triflate. More preferably, the leaving group is imidazole or triazole or derivatives thereof, chloride, trichloromethanol, N-hydroxysuccinimide or a derivative thereof, or a (substituted) phenol. More preferably, the leaving group is an imidazole, a N-hydroxysuccinimide, a (substituted) phenol or a chloride. Even more preferred are imidazole or N-hydroxysuccinimide; most preferred is imidazole.

Note that, for convenience reasons only, leaving groups are mentioned interchangeably in this application as acids (phenol and not phenolate; imidazole and not imidazolyl anion) or as bases (chloride and not hydrochloride).

In one embodiment of this invention, LG₁ and LG₂ are the same leaving group and preferred examples of the (thio)carbonyl containing reactants are then 1,1'-carbonyldiimidazole, 1,1'-carbonylbis(2-methytimidazole), 1,1'-carbonylbis(benzotriazole), 1,1'-carbonyl-di-(1,2,4-triazole), N,N'-disuccinimidyl carbonate, diphenyl carbonate, di-para-nitrophenyl carbonate, di-pentafluorophenyl carbonate, di-2,3,5,6-tetrafluorophenyl carbonate, di-methylphenyl carbonates, di-methoxyphenyl carbonates, triphosgene, phosgene, thiophosgene, 1,1'-thiocarbonyldiimidazole, and the like. More preferred examples are 1,1'-carbonyldiimidazole, N,N'-disuccinimidyl carbonate, diphenyl carbonate, di-para-nitrophenyl carbonate, di-pentafluorophenyl carbonate, and di-para-methylphenyl carbonate. Even more preferred are 1,1'-carbonyldiimidazole and N,N'-disuccinimidyl carbonate, most preferred is 1,1'-carbonyldiimidazole.

In another embodiment of this invention, LG₁ and LG₂ are different leaving groups, so that the reactant is a-symmetrical. In this case, LG₁ is preferably a halogen or N-hydroxysuccinimide, most preferably a chloride. Additionally, LG₂ is preferably a (substituted) phenol or an alcohol such as 2-halo, 2,2-dihalo, 2,2,2-trihalo, 2-methylsulfonyl alcohols, or (substituted) benzyl alcohols. More preferably, LG₂ is a (substituted) phenol such as preferably phenol, (ortho, para or metha) halo-, methoxy- or nitro-phenol, most preferably LG₂ is phenol. In this embodiment, specific examples of (thio)carbonyl containing reactants are therefore chloroformates, such as (substituted) phenyl chloroformates, (substituted) benzyl chloroformates or 2,2,2-trichloroethyl chloroformate, or N-succinimidyl carbonates such as N-succinimidyl-2,2,2-trichloroethyl carbonate, N-(benzyloxycarbonyloxy)-succinimide and 2-(methylsulfonyl)-ethyl-N-succinimidyl carbonate. More preferred examples are (substituted) phenyl chloroformates, most preferably phenyl-chloroformate.

In another less preferred and specific embodiment of this invention, the (thio)carbonyl containing reactant is not a (thio)carbonate derivative, as those mentioned above, but is an oxalic acid derivative. Examples are oxalylhalides such as oxalyl chloride, N,N'-disuccinimidyl oxalate, 1,1'-oxalyldiimidazole, (sulfosuccinimidyl)oxalate sodium salt, di(1-benzotriazolyl) oxalate and di((substituted) phenyl) oxalates.

The diamine that is used in Reactions (ii) and (iv) can be any diamine known in the art. L is defined as above and Rs₁ and Rs₂ are residues that are independently selected from the group consisting of hydrogen, cyclic, linear or branched C₁ - C₂₀ alkyl groups, C₆ - C₂₀ aryl groups, C₇ - C₂₀ alkaryl groups and C₇ - C₂₀ arylalkyl groups, wherein the alkyl groups, aryl groups, alkaryl groups and arylalkyl groups optionally comprise 1 - 5 heteroatoms selected from the group consisting of oxygen, nitrogen and sulphur, and where Rs₁ and Rs₂ may optionally be linked with each other, thereby forming a cyclic alkylene, arylene, alkarylene or arylalkylene structure. More preferably, Rs₁ and Rs₂ are residues that are independently selected from the group consisting of hydrogen, cyclic, linear or branched C₁ - C₁₂ alkyl groups, and C₆ - C₁₂ aryl groups, wherein the alkyl groups or the aryl groups optionally comprise 1 - 3 oxygens, and where Rs₁ and Rs₂ may optionally be linked with each other, thereby forming a cyclic alkylene or arylene structure. Even more preferably, Rs₁ and Rs₂ are independently selected from hydrogen, and linear or branched C₁ - C₆ alkyl groups, where Rs₁ and Rs₂ may optionally be linked with each other thereby forming a cyclic alkylene structure. More preferably, Rs₁ and Rs₂ are independently selected from hydrogen and linear or branched C₁ - C₄ alkyl groups, most preferably Rs₁ and Rs₂ are hydrogens.

According to the above, the diamine may have two primary amine groups (Rs₁ and Rs₂ are hydrogen), two secondary amine groups (Rs₁ and Rs₂ are not hydrogen) or one primary amine group and one secondary amine group (either Rs1 or Rs2 is a hydrogen). Primary amines are preferred due to their higher reactivity.

Examples of diamines used in Reactions (ii) and (iv) comprise - but are not limited to - alkylene primary diamines such as 1,4-diaminobutane, 1,6-diaminohexane, 1,8-diaminooctane, 1,10-diaminodecane, 1,12-diaminododecane, 2,2'-oxydiethylamine, isophorone diamine, 1,8-diamino-p-menthane, 2,2,4-(2,4,4)-trimethyl-1,6-hexanediamine, 4,4'-methylene-bis(cyclohexyl amine) and lysine or derivatives thereof, such as acid protected lysines, secondary diamines such as piperazine, 2-methyl piperazine, 2,6-dimethylpiperzine, N,N'-dimethyl-1,6-hexanediamine, N,N'-dibutyl-1,6-hexanediamine and 4,4'-bipiperidine, diamines with a primary amine and secondary amine group such as N-alkyl-1,6-diaminohexanes or 2-amino-pyrrolidine or 3-amino-piperidine, aromatic diamines such as metha- and para-diaminobenzenes, metha- and para-diaminotoluenes, metha- and para-aminobenzylamines. Preferred diamines are alkylene diamines with more than 3, preferably more than 5 carbon atoms in their constitution (i.e. more hydrophobicity is introduced), such as 1,4-diaminobutane, 1,6-diaminohexane, 1,8-diaminooctane, 1,10-diaminodecane, 1,12-diaminododecane, isophorone diamine, 1,8-diamino-p-menthane, 2,2,4-(2,4,4)-trimethyl-1,6-hexanediamine, 4,4'-methylene-bis(cyclohexyl amine), lysine and derivatives thereof, N,N'-dimethyl-1,6-hexanediamine and 4,4'-bipiperidine. More preferred diamines are 1,6-diaminohexane, 1,10-diaminodecane, 1,12-diaminododecane and isophorone diamine. Even more preferred examples are 1,12-diaminododecane and 1,6-diaminohexane. Most preferred example is 1,6-diaminohexane.

Specifically, 1,2-diamino-arylenes, 1,2-ethylenediamines (L=C₂) and 1,3-propylenediamines (L=C₃) are less preferred, as these diamines lead to prepolymers with a lesser stability.

Reactions (i) and (iii) can be executed in the bulk or in solution, preferably in concentrated solution (molar concentration of the (thio)carbonyl containing reactant in the reaction mixture is higher than 0.1 M, preferably higher than 0.3 M, most preferably higher than 0.5 M). Reaction solvents are preferably aprotic solvents known in the art, such as ethers, such as diethyl ether, THF, methyl-tetrahydrofuran, dioxane and methyl-tert-butyl ether, DMSO, dimethyl acetamide, NMP, chloroform, dichloromethane, ketones such as acetone, MEK and methyl-tert-butyl ketone, esters such as ethyl acetate and butyl acetate, toluene, alkanes such as cyclohexane, pentane, hexane and isododecane, and cyclomethicones such as D5.

Reaction (i) and (iii) are preferably executed at temperatures lower than 130 °C, more preferably lower than 70 °C, most preferably lower than 40 °C.

The hydroxy functional polymer P-(OH)ₙ is preferably used dry or dried, i.e. containing no or little water. Preferably, the reaction is executed under an inert atmosphere of nitrogen or argon.

The (thio)carbonyl containing reactant is preferably used in molar excess when compared to the moles of hydroxy groups in the reactant P-(OH)ₙ. Preferably, the molar excess is higher than 1.2-fold, more preferably, higher than 1.5-fold, most preferably higher than 2-fold. In case LG₂ is different than LG₁, the (thio)carbonyl containing reactant is preferably used in slight molar excess. Preferably, the molar excess is lower than 1.5-fold, more preferably, lower than 1.25-fold, most preferably lower than 1.1-fold. In case chloroformates are used as (thio)carbonyl containing reactants (LG₁ = Cl), a base is used to scavenge the formed HCl (i.e. H·LG₁). Preferred examples of such bases are pyridines and tertiary amines such as triethylamine or other trialkylamines.

The activated polymer can be isolated by different type of work-up procedures that are known in the art such as precipitation, extraction, washing, drying, filtration and evaporation procedures, or combinations thereof Before work-up, the excess of (thio)carbonyl containing reactant may be eliminated. Elimination of the (thio)carbonyl containing reactant can be done by reaction with water or with an alcohol, preferably a secondary alcohol such as for example isopropanol or 2,3-butanediol. Most preferably elimination is done with water. Finally, the reaction mixture of reaction (i) or (iii) can be used in the next reaction step (ii) or (iv) without work-up, preferably after elimination of the excess of (thio)carbonyl containing reactant.

Reactions (ii) and (iv) can be executed in the bulk or in solution, preferably in concentrated solution (molar concentration of the diamine reactant in the reaction mixture is higher than 0.1 M, preferably higher than 0.3 M, most preferably higher than 0.5 M). Reaction solvents are preferably aprotic solvents known in the art, such as ethers, such as diethyl ether, THF, methyl-tetrahydrofuran, dioxane and methyl-tert-butyl ether, DMSO, dimethyl acetamide, NMP, chloroform, dichloromethane, toluene, alkanes such as cyclohexane, pentane, hexane and isododecane, and cyclomethicones such as D5. Esters such as ethyl acetate and butyl acetate are less preferred; ketones such as acetone, MEK and methyl-tert-butyl ketone are even less preferred.

Reactions (ii) and (iv) are preferably executed at temperatures lower than 130 °C, more preferably lower than 70 °C, most preferably lower than 40 °C.

The diamine is preferably used in molar excess when compared to the moles of activated species O-C(Q)-LG₂ in the activated polymer reactant. Preferably, the molar excess of diamine is higher than 1.5-fold, more preferably, higher than 2-fold, most preferably higher than 3-fold. Importantly, in reactions (ii) and (iv), the activated polymer is preferably added in portions to the excess of diamine. This can for example be done by drop wise addition of a solution of the activated polymer to a solution of the diamine.

The amine functional polymer can be isolated by different type of work-up procedures that are known in the art such as precipitation, extraction, washing, drying, filtration and evaporation procedures, or combinations thereof For some low-volatile diamines such as piperazine or 1,3-propylene amine, the excess of diamine can be removed by evaporation.

The amine functional polymers as prepared in Reactions (ii) and (iv) preferably have a high degree of definition meaning that, (a) on average at least 65% of the alcohol groups in the alcohol functional polymer P-(OH)ₙ precursors are converted into amine groups, more preferably at least 75%, most preferably at least 85%, and that (b) on average at least 80% of all functional end groups in the amine functional polymer are indeed amine groups, more preferably more than 90%, most preferably more than 95%, and that (c) the average number molecular weight of the amine functional polymer does not exceed by more than 50% the summation of the number molecular weight of the alcohol functional polymer P-(OH)ₙ plus n times the molecular weight of the diamine, more preferably by more than 40%, most preferably by more than 30%.

### THE HYDROGEL

In an embodiment of this invention, the water gellant comprises biodegradable moieties, that may be single bonds (e.g. an ester bond) or linkages or may be regions with multiple biodegradable bonds (e.g. a polyester or oligoester). The moieties may occur within parts of the polymer backbone P, in the linker L, in the simple hydrogen bonding units, and/or in the attachments between the polymer P and the linker L, or the attachments between the linker L and the simple hydrogen bonding units. Examples of biodegradable bonds or linkages are covalent ester, ortho-ester, ortho-ester, urethane, thio-urethane or amide bonds, but are preferably ester bonds.

The amount of the water gellant in the hydrogel ranges from 0.3% - 50.0% by weight, preferably from 1% - 25% by weight, more preferably from 1% - 15% by weight, more preferably from 1% - 8% by weight, and most preferably from 1% - 3.4% by weight, based on the total weight of the hydrogel. The hydrogel may contain additional functional ingredients that will contribute to the specific use of the hydrogel.

The hydrogel comprises 50.0 to 99.7 wt.% of water, preferably 60 to 99 wt.%, and most preferably 80 to 98 wt.%, based on the total weight of the hydrogel.

Obviously, but not preferably, the hydrogel may contain other polar solvents, preferably those solvents having a dielectric constant s at 20°C of at least about 20. The upper limit is given by the dielectric constant ε at 20 °C for pure water which is about 80 (Handbook of Chemistry & Physics, 59th Ed., page E-61, 1978 - 1979). Suitable examples are DMSO, alcohols, preferably ethanol and glycols, but more preferably ethanol and ketones, such as acetone. Preferably, the amount of other solvents is lower than 15% by weight, more preferably lower than 8% by weight, and most preferably lower than 2% by weight, calculated on the basis of the total weight of the hydrogel.

The hydrogels according to the present invention have a wide range of mechanical properties, ranging from elastic to tough, depending on the nature of the polymer backbone P, the nature of the linker L, and the number of simple hydrogen bonding units attached to the polymer. In the preferred case of elastic hydrogels, the hydrogel materials preferably display an elongation at break at 25°C greater than about 5%, more preferably greater than 20%, more preferably greater than 100%.

The reversible cross-links in the hydrogel allow for switching the gel into a liquid by the application of heat, changing the nature of the solvent or the concentration of the water gellant. Consequently, the processing or administration of the hydrogel can be done with processes known for liquids, like spraying or pumping. Preferably, the polymers present in the hydrogel have a relatively low number average molecular weight, preferably in the range from 1200 to 100000, more preferably from 2000 to 50000, most preferably from 7500 to 21000, in order to allow for easy solution processing of the water gellant, such as pumping or spraying.

The hydrogels can for example be used in pharmaceutical applications or biomedical applications such as controlled drug-delivery, tissue engineering, wound-dressings and wound-care, artificial articular cartilage material, soft contact lenses, tissue-adhesion, lubricating coatings for medical devices, as superabsorbers, and as thickeners for aqueous solvents.

The hydrogels according to the present invention can be prepared by three different methods: (i) the hydrogel can be dissolved in aqueous solvent at elevated temperatures between 40°C and 95°C, preferably between 60°C and 90°C, followed by cooling down to temperatures between 0°C and 40°C, preferably between 20°C and 40°C; (ii) the hydrogel can be swelled in aqueous solvent at temperatures between 0°C and 60°C, preferably between 20°C and 40°C; and (iii) the hydrogel can be dissolved or dispersed in any organic water-miscible solvent, such as for example THF, acetone, MEK, alcohols, such as ethanol, DMSO, NMP, or solvent mixture, followed by the addition of water and subsequently the optional removal of the organic solvent with evaporation in vacuo or removal of the organic solvent by washing with water.

The hydrogels are applied in their gelled form after gelling the aqueous solvent with water gellant in a mould, eventually followed by cutting of the gel, or the water gellants are applied in their liquid state followed by gelling the polymers to a hydrogel after administration of the effective amount of the polymer to the desired site.

Additional ingredients in the hydrogels can be antioxidants, preservatives, fillers, salts, pH-buffers, dyes, bone-extracts, or bioactive species.

In another preferred embodiment of this invention, the hydrogels are used in biomedical applications, such as carriers for the controlled release of drugs, scaffolds for tissue-engineering, or as adhesives or sealants for tissue. In these uses, the hydrogels also preferably comprise a biologically active or pharmaceutically active compound. The hydrogel may also comprise a bioactive species, e.g. a living cell, an enzyme, or a micro-organism. A living cell in this embodiment means and includes individual animal and plant cells, cell clusters, tissues, organs and organisms, including organisms such as bacteria, fungi or moulds. A biologically active or pharmaceutically active compound, as used herein, includes a compound which provides a therapeutic, diagnostic, or prophylactic effect, a compound that affects or participates in tissue growth, cell growth, cell differentiation, a compound that may be able to invoke a biological action such as an immune response, or could play any other role in one or more biological processes. Such compounds, peptide or non-peptide, protein or non-protein, include, but are not limited to, antimicrobial agents (including antibacterial, hemotherapeutic and anti-fungal agents), anti-viral agents, anti-tumor agents, hormones, hormone antagonistics, corticosteroids such as mineralocorticosteroids or glucocorticosteroids, androgents, estrogens, progestins immunogenic agents, antiinflammatory agents, anti-gout agents, centrally acting analgesics, local anesthetics, centrally active muscle relaxants, growth factors, (fluorescent) dyes, contrast agents, nucleic acids, lipids, lipopolysaccharides, (poly)saccharides, vitamins, and peptides, polypeptides and proteins in general.

Apart from the previous list, it is also possible to load the hydrogel with inorganic compounds, such as reactive oxygen scavengers and bone-extracts like apatite.

It is possible within the scope of the present invention to incorporate drugs of a polymeric nature, but also to incorporate drugs or vitamins of a relatively small molecular weight of less than 1500, or even less than 500.

Additionally, two or more different biologically active compounds may be present in the hydrogel. This is especially beneficial when the bioactivity is based on multivalent and/or synergistic interactions. A non-limiting example of such interaction is that cell adhesion is advantageously mediated by a combination of RGD and PHSRN peptides.

### EXAMPLES

The following examples further illustrate preferred embodiments of the invention. When not specifically mentioned, chemicals are obtained from Aldrich. The synthesis with simple hydrogen bonding unit building blocks is shown, as well as the synthesis of amine functional prepolymers with hydrophobic endgroups, and the synthesis of the water gellants. Finally, the hydrogels of this invention are introduced, by explaining their preparation and showing their properties.

Examples A, B and C are not according to the invention.

### Example 1: Preparation of UPyl

1,6-Hexyldiisocyanate (650 g) and methylisocytosine (or 2-amino-4-hydroxy-6-methylpyrimidine, 65.1 g) were suspended in a 2-liter flask. The mixture was stirred overnight at 100 °C under an argon atmosphere. After cooling to room temperature, a liter of pentane was added to the suspension, while stirring was continued. The product was filtered, washed with several portions of pentane and dried in vacuum. A white powder was obtained. ¹H NMR (400 MHz, CDCl₃): δ 13.1 (1H), 11.8 (1H), 10.1 (1H), 5.8 (1H), 3.3 (4H), 2.1 (3H), 1.6 (4H), 1.4 (4H). FT-IR (neat): v (cm⁻¹) □2935, 2281, 1698, 1668, 1582, 1524, 1256.

### Example A: Preparation of PEG2000-diamine prepolymer with n-C₁₀ linker L

Reaction (i): To a solution of 1,1'-carbonyldiimidazole (CDI) (8.125g, 40 mmol) in CHCl₃ (100 mL) was added over a period of 5 minutes a solution of dried telechelic hydroxy functional polyethylene glycol (PEG2000 diol, 10g, 5 mmol, MW=2.0 kDalton) in CHCl₃ (100 mL). The reaction was executed under an argon atmosphere and was allowed to stir at room temperature for 24 hours. After concentration to a total volume of 100 mL via rotary evaporation, the solution was diluted slowly with diethyl ether until a total volume of ∼600-800mL was reached, resulting in precipitation of the activated polymer. This dilution-precipitation procedure was repeated to ensure complete removal of excess CDI prior to moving to the next reaction (ii). The CDI-activated material was moved directly into the next step to avoid possible hydrolysis of the end group. -

Reaction (ii): To a solution of 1,10-diaminodecane (3.21 g, 18.6 mmol) in CHCl₃ (150 mL) was added slowly, over a one hour period, a solution of the CDI activated PEG-material as prepared in the first reaction (5 g, 2.3 mmol, MW=2.2 kD with activated endgroups) in CHCl₃ (75 mL). The reaction was allowed to stir overnight at room temperature under an argon atmosphere. After concentration of the solution to a volume of about 75 mL, diethyl ether was added slowly until a total volume of ∼600-800 mL was reached, resulting in precipitation of the polymer. This dilution-precipitation procedure was repeated to ensure complete removal of excess diamine. ¹H NMR (400 MHz, CDCl₃): δ 4.85 (2H), 4.2 (4H), 3.8-3.4 (EO-units), 3.15 (4H), 2.7 (4H), 2.0 (4H, br), 1.45 (8H), 1.25 (20H). This material can be denoted as 2K-10, where 2K stands for the PEG2000 polymer backbone and 10 stands for the n-decyl linker.

### Example B: Preparation of PEG-diamines with incorporated linkers L

Similar procedures to the one shown in example A have been followed for different molecular weights of hydroxy functional telechelic polyethylene glycols (PEGs 2 kD to 35 kD), for another hydroxy functional telechelic polymer (PCL1250, polycaprolactone) and for different diaminoalkanes. See Table 1 below for prepared materials and for a brief characterization of these materials.

**Table 1: Prepared telechelic PEGs with amine functional linkers L as endgroups**

| **Example** | **PEG-polymer^{a}** | **Diamine** | **L** | **Material** |
|---|---|---|---|---|
| **2K-10** | PEG2000 | 1,10-diaminodecane | n-C10 | White stable powder |
| **2K-6** | PEG2000 | 1,6-diaminohexane | n-C6 | White stable powder |
| **3K-6** | PEG3000 | 1,6-diaminohexane | n-C6 | White stable powder |
| **3K-12** | PEG3000 | 1,12-diaminododecane | n-C12 | White stable powder |
| **6K-2** | PEG6000 | 1,2-diaminoethane | n-C2 | Unstable material |
| **6K-4** | PEG6000 | 1,4-diaminobutane | n-C4 | White stable powder |
| **6K-6** | PEG6000 | 1,6-diaminohexane | n-C6 | White stable powder |
| **6K-8** | PEG6000 | 1,8-diaminohexane | n-C8 | White stable powder |
| **6K-10** | PEG6000 | 1,10 diaminodecane | n-C10 | White stable powder |
| **6K-12** | PEG6000 | 1,12-diaminododecane | n-C12 | White stable powder |
| **10K-8** | PEG10000 | 1,8-diaminohexane | n-C8 | White stable powder |
| **10K-10** | PEG10000 | 1,10-diaminodecane | n-C10 | White stable powder |
| **10K-10c^{b}** | PEG10000 | isophorone diamine | cyclic and branched C10 | White stable powder |
| **10K-12** | PEG10000 | 1,12-Dodecanediamine | n-C12 | White stable powder |
| **20K-10** | PEG20000 | 1,10-diaminodecane | n-C10 | White stable powder |
| **20K-12** | PEG20000 | 1,12-diaminododecane | n-C12 | White stable powder |
| **35K-10** | PEG35000 | 1,10-diaminodecane | n-C10 | White stable powder |
| **35K-12** | PEG35000 | 1,12-diaminododecane | n-C12 | White stable powder |
| **PCL-6^{c}** | PCL1250 | 1,6-diaminohexane | n-C6 | Wax |

| | | | | |
|---|---|---|---|---|
| ^{a} The number denotes the molecular weight of the PEG starting polymer. ^{b} The reaction (ii) has been executed at about 60 °C in stead of room temperature. ^{c} PCL-6 and its CDI-activated precursor was not isolated by precipitation in ether, but by extraction into chloroform using a brine water layer. | | | | |

It is clear from the Table that all PEG-based materials are stable white powders, apart from the ethylene diamine end capped material 6K-2. Possibly, the end group forms cyclic ethylene urea, thereby reforming the alcohol end group.

### Example C: Preparation of telechelic water gellants with 4H-units UPy1

**UPy1** (585 mg; 2.0 equivalents) was added as a powder to a solution of pre-dried polyethylene glycol 10.000 terminated with aminododecyl groups (10.3g; entry 10K-12 in Table 1) in CHCl₃ (50 mL). The reaction was allowed to stir overnight under an argon atmosphere at an oil bath temperature of 70 °C. After cooling down to room temperature, ethanol was added (100 mL), and the hazy mixture was filtered over celite. The clear filtrate was concentrated by vacuum evaporation, and was diluted with ether under stirring. A white precipitate formed, and the mixture was stirred for 20 mins, then allowed to settle prior to filtration and washing with excess ether. The product was vacuum dried to give a white powder (95% yield). ¹H NMR (400 MHz, CDCl₃): δ 13.1 (2H), 11.8 (2H), 10.1 (2H), 5.8 (2H), 4.9 (2H), 4.7 (2H), 4.5 (2H), 4.2 (4H), 3.8-3.3 (EO-units), 3.3 (4H), 3.1 (12H), 2.2 (6H), 1.6-1.1 (56H).
The powder and distilled water were added to a vial (10% gellant loading) and mixed with vigorous stirring at ca. 55-60 °C for 1 to 2 hours. The polymer solution was removed from the water bath and placed on the bench at room temperature to set up over 24-48 hrs. An elastic hard gel had formed.
Method A: the selected gellant from Table 1 was mixed with the desired amount of water and stirred at 55 °C for 12 hours resulting in a homogeneous solution that gelled upon cooling to 25 °C. A 24-hour equilibration time was observed.

### Example 2: Preparation of telechelic water gellants with urea units

In similar reaction conditions as those described in Example C, PEG6000 modified with aminodecyl groups (entry 6K-10 in Table 1) has been end capped with dodecylmonoisocyanate (C12-isocyanate). The material was isolated as a white powder. ¹H NMR (400 MHz, CDCl₃): δ 4.85 (2H), 4.4 (4H), 4.2 (4H), 3.8-3.4 (EO-units), 3.15 (12H), 1.5 (12H), 1.3-1.1 (60H), 0.9 (6H).

### Example 3: Preparation of chain extended water gellants with urea units

1,12-Dodecyldiisocyanate (0.12 g; ca. 1.0 equivalent) was added to a solution of PEG6000 modified with aminodecyl groups (3g; entry 6K-10 in Table 1) in CHCl₃ (50 mL). The reaction was allowed to stir overnight at room temperature. FT-IR showed the disappearance of the isocyanate. The CHCl₃ solution was concentrated until a noticeable viscosity increase was observed, then precipitation was induced by dilution with diethyl ether under stirring, halting at a ca. 5:1 ether/mother solution ratio. The precipitation mixture was stirred for 20 mins, then allowed to settle prior to filtration and washing with excess ether. The product was dried giving a yield of 95% of a white elastic material. ¹H NMR (400 MHz, CDCl₃): δ 4.85, 4.5, 4.4, 4.2, 3.8-3.4 (EO-units), 3.15, 1.5, 1.3-1.1.

### Examples 4: Preparation of urea-type water gellants and their hydrogel properties

More telechelic and chain extended urea-type water gellants have been prepared than the ones introduced in Examples 2 and 3, and similar synthetic methods as those described there have been used to prepare them. Table 2 shows the two components of these gellants, typifies the hydrogel properties prepared from water and the chosen solids loading of the gellant. Method A of gel preparation has been used.

**Table 2: Prepared urea-type water gellants and their hydrogel properties**

| **Water Gellant** | **Prepolymer from example** | **Isocyanate building block** | **Method of gel preparation** | **Solids Loading ^{a}** | **Hydrogel properties** |
|---|---|---|---|---|---|
| **11A** | 6K-10 | C12-NCO | A | 7% | soft |
| **11A** | 6K-10 | C12-NCO | A | 12% | elastic |
| **11B** | 10K-10 | C12-NCO | A | 7% | tough gel |
| **11B** | 10K-10 | C12-NCO | A | 12% | brittle gel |
| **11C** | 20K-10 | C12-NCO | A | 11% | elastic gel |
| **11D** | 35K-10 | C12-NCO | A | 7% | sticky gel |
| **11D** | 35K-10 | C12-NCO | A | 15% | tough gel |
| **12A** | 6K-10 | C12-(NCO)2 | A | 7% | elastic gel |

| | | | | | |
|---|---|---|---|---|---|
| a: solids loading is the weight of water gellant divided by the total weight of the hydrogel. | | | | | |

## Claims

1. A hydrogel comprising:
(a) 0.3 - 50.0 wt.%, based on the total weight of the hydrogel, of a water gellant, wherein the water gellant is obtainable by converting a hydrophilic polymer backbone P, wherein P is selected from polyethers, polyesters, or a copolymer of such polymers, in a first step into an amine functional prepolymer according to the formula P-[L]ₙ, wherein in a second step a simple hydrogen bonding unit is introduced at the termini of the hydrophobic linker L by reacting the amine functional prepolymer P-[L]ₙ with a mono- or difunctional compound selected from the group consisting of (di)isocyanates, (di)thioisocyanates, (di)amines which are optionally activated, (di)acid chlorides, (di)esters which are optionally activated, (di)ols which are optionally activated, (di)acryl amides, (di)methacryl amides, and (di)thiols which are optionally activated; wherein P and L are connected to each other by moieties selected from the group consisting of (thio)urea, (thio)urethane, amide, ester, carbonate, secondary amine, tertiary amine and ether moieties,
wherein:
n is in the range of 1.8 to 10;
L is a hydrophobic linker selected from the group consisting of cyclic, linear or branched C₂ - C₂₄ alkylene groups, C₆ - C₂₄ arylene groups, C₇ - C₂₄ alkarylene groups and C₇ - C₂₄ arylalkylene groups, wherein the alkylene groups, arylene groups, alkarylene groups and arylalkylene groups optionally, but not preferably, comprise 1 - 5 heteroatoms selected from the group consisting of O, N and S; and
(b) 50.0 to 99.7 wt.% water.

2. The hydrogel according to Claim 1, wherein the mono- or difunctional compound is selected from the group consisting of (di)isocyanates, (di) activated amines and (di) activated esters.

3. The hydrogel according to Claim 1 or Claim 2, wherein the mono- or difunctional compound is selected from the group consisting of (di)isocyanates.

4. The hydrogel according to any one of Claims 1 - 3, wherein the simple hydrogen bonding unit is selected from the group consisting of thio(urea), amide and (thio)urethane.

5. The hydrogel according to any one of Claims 1 - 4, wherein the hydrogel comprises a water gellant that is obtainable without the use of a toxic metal-based catalyst.

6. The hydrogel according to any one of Claims 1 - 4, wherein the linker L is a diamine and wherein the hydrophilic polymer backbone P has the formula P-(OH)ₙ, wherein n is in the range of 1.8 to 10.

7. The hydrogel according to any one of Claims 1 - 5, wherein the water gellant has a molecular weight of 1200 to 1.000.000.

8. The hydrogel according to any one of Claims 1 - 6, wherein the hydrophilic polymer backbone P has a molecular weight of 250 to 200.000.

9. The hydrogel according to any one of Claims 1 - 7, wherein the hydrophilic polymer backbone P is telechelic.

10. The hydrogel according to any one of Claims 1 - 8, wherein the hydrophilic polymer backbone P has the formula P-(OH)₂.

11. The hydrogel according to Claim 9 or Claim 10, wherein the hydrophilic polymer backbone P-(OH)₂ is a polyethylene glycol.

12. The hydrogel according to any one of Claims 1 - 7, wherein the functional grafted hydrophilic polymer backbone P-(OH)ₙ is prepared from polymers obtainable by (co) polymerization of vinyl monomers, the vinyl monomers being selected from the group consisting of
(a) monomers according to the formula (8) 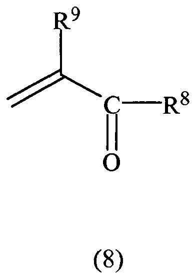 wherein R⁸ is independently selected from the group consisting of:
(i) OH;
(ii) C₁ - C₁₂ linear or branched alkoxy, optionally substituted with 1 - 6 hydroxy groups;
(iii) amide according to the formula -N(R¹⁰)₂ wherein R¹⁰ can be hydrogen or C₁ - C₆ linear or branched alkyl, optionally substituted with 1 - 6 hydroxy groups;
(iv) ammonium salt according to the formula -[N(R¹⁰)₃]⁺X⁻, wherein R¹⁰ is as defined for (iii) and X is a halogen atom; and
(v) a group according to the formula (9) 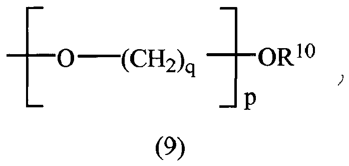 wherein R¹⁰ is as defined for (iii) and p is 1 - 50 and q = 2 or 3; and
wherein R⁹ is hydrogen or methyl;
(b) C₁ - C₁₂ linear or branched alkyl vinyl ether;
(c) vinyl alcohol;
(d) C₂ - C₁₂ α-alkenylene ω-sulfonate having an alkaline earth metal cation or an alkali metal cation;
(e) C₇ - C₁₂ vinylaryl sulfonate according to formula (10) 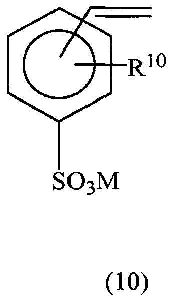 wherein R¹⁰ is as defined for (iii) and M is an alkaline earth metal or an alkali metal cation;
(f) CH₂=CH-R¹¹, wherein R¹¹ is selected from the group consisting of pyrrolyl, imidazolyl, pyrazolyl, pyridinyl, pyrazinyl, pyrimidinyl, pyrrolidyl, indolyl,
indolizinyl, indazolyl, purinyl, quinolizinyl, quinolinyl, isoquinolinyl, phthalizinyl, naphtypyridinyl, quinoxalinyl, quinazolinyl, cinnolinyl, pteridinyl, pyrrolidonyl, pyrrolinyl, pyrrolidinyl, imidazolidinyl, imidazolinyl, pyrazolidinyl, pyrazolinyl, piperidyl, piperazinyl, indolinyl, isoindolinyl, morpholinyl, isoxazolyl, furazinyl, and isothiazolyl;
(g) CH₂=CH-O-C(O)R¹², wherein R¹² is selected from the group consisting of C₁ - C₆ linear or branched alkyl;
(h) CH₂=CH-CH₂OR¹³, wherein R¹³ is selected from the group consisting of hydrogen and C₁ - C₆ linear or branched alkyl; and
(i) N-vinyl lactams according to the formula (11) 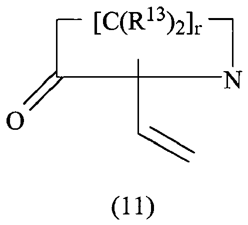 wherein R¹³ is as defined for (h) and r is 2 - 6.

13. The hydrogel according any one of the preceding claims, wherein the hydrogel comprises a biologically active or pharmaceutically active compound or a bioactive species.

14. Use of the hydrogel according to any one of claims 1 - 12 in pharmaceutical or biomedical compositions.

15. A process for preparing a hydrogel according to any one of Claims 1 - 12, wherein:
(a) the hydrogel is dissolved in an aqueous solvent at a temperatures between 40°C and 95°C, followed by cooling down to temperatures between 0°C and 40°C;
(b) the hydrogel is swelled in an aqueous solvent at a temperature between 0°C and 60°C; or
(c) the hydrogel is dissolved or dispersed in an organic water-miscible solvent or solvent mixture, followed by the addition of water and subsequently the optional removal of the organic solvent with evaporation in vacuo or removal of the organic solvent by washing with water.

## Patentansprüche

1. Hydrogel, umfassend:
(a) 0,3 bis 50,0 Gew.-%, bezogen auf das Gesamtgewicht des Hydrogels, eines Wassergeliermittels, wobei das Wassergeliermittel dadurch erhältlich ist, dass man ein hydrophiles Gerüst P, wobei P aus der Gruppe ausgewählt ist, die aus Polyethern, Polyestern und Copolymeren solcher Polymere besteht, in einem ersten Schritt in ein aminfunktionelles Prepolymer gemäß der Formel P-[L]ₙ umwandelt, wobei in einem zweiten Schritt eine einfache Wasserstoffbrückeneinheit an den Enden des hydrophoben Linkers L eingeführt wird, indem man das aminfunktionelle Prepolymer P-[L]ₙ mit einer mono- oder difunktionellen Verbindung umsetzt, die aus der Gruppe ausgewählt ist, die aus (Di)isocyanaten, (Di)thioisocyanaten, (Di)aminen, die gegebenenfalls aktiviert sind, (Di)säurechloriden, (Di)estern, die gegebenenfalls aktiviert sind, (Di)olen, die gegebenenfalls aktiviert sind, (Di)acrylamiden, (Di)methacrylamiden und (Di)thiolen, die gegebenenfalls aktiviert sind, besteht; wobei P und L über Struktureinheiten, die aus der Gruppe ausgewählt sind, die aus (Thio)harnstoff-, (Thio)urethan-, Amid-, Ester-, Carbonat-, sekundären Amin-, tertiären Amin- und Ether-Struktureinheiten besteht, miteinander verbunden sind;
wobei:
n im Bereich von 1,8 bis 10 liegt;
L ein hydrophober Linker ist, der aus der Gruppe ausgewählt ist, die aus cyclischen, linearen oder verzweigten C₂-C₂₄-Alkylengruppen, C₆-C₂₄-Arylengruppen, C₇-C₂₄-Alkarylengruppen und C₇-C₂₄-Aryl-alkylengruppen besteht, wobei die Alkylengruppen, Arylengruppen, Alkarylengruppen und Arylalkylengruppen gegebenenfalls, aber nicht vorzugsweise, 1 bis 5 Heteroatome umfassen, die aus der Gruppe ausgewählt sind, die aus O, N und S besteht; und
(b) 50,0 bis 99,7 Gew.-% Wasser.

2. Hydrogel gemäß Anspruch 1, wobei die mono- oder difunktionelle Verbindung aus der Gruppe ausgewählt ist, die aus (Di)isocyanaten, (di)aktivierten Aminen und (di)aktivierten Estern besteht.

3. Hydrogel gemäß Anspruch 1 oder 2, wobei die mono- oder difunktionelle Verbindung aus der Gruppe ausgewählt ist, die aus (Di)isocyanaten besteht.

4. Hydrogel gemäß einem der Ansprüche 1 bis 3, wobei die einfache Wasserstoffbrückeneinheit aus der Gruppe ausgewählt ist, die aus (Thio)harnstoff, Amid und (Thio)urethan besteht.

5. Hydrogel gemäß einem der Ansprüche 1 bis 4, wobei das Hydrogel ein Wassergeliermittel umfasst, das ohne Verwendung eines auf einem toxischen Metall basierenden Katalysators erhältlich ist.

6. Hydrogel gemäß einem der Ansprüche 1 bis 4, wobei der Linker L ein Diamin ist und wobei das hydrophile Polymergerüst P die Formel P-(OH)ₙ aufweist, wobei n im Bereich von 1,8 bis 10 liegt.

7. Hydrogel gemäß einem der Ansprüche 1 bis 5, wobei das Wassergeliermittel ein Molekulargewicht von 1200 bis 1 000 000 aufweist.

8. Hydrogel gemäß einem der Ansprüche 1 bis 6, wobei das hydrophile Polymergerüst P ein Molekulargewicht von 250 bis 200 000 aufweist.

9. Hydrogel gemäß einem der Ansprüche 1 bis 7, wobei das hydrophile Polymergerüst P telechel ist.

10. Hydrogel gemäß einem der Ansprüche 1 bis 8, wobei das hydrophile Polymergerüst P die Formel P-(OH)₂ aufweist.

11. Hydrogel gemäß Anspruch 9 oder 10, wobei das hydrophile Polymergerüst P-(OH)₂ ein Polyethylenglycol ist.

12. Hydrogel gemäß einem der Ansprüche 1 bis 8, wobei das funktionelle gepfropfte hydrophile Polymergerüst P-(OH)ₙ aus Polymeren hergestellt wird, die durch (Co)polymerisation von Vinylmonomeren erhältlich sind, wobei die Vinylmonomeren aus der Gruppe ausgewählt sind, die aus folgenden besteht:
(a) Monomeren gemäß Formel (2) 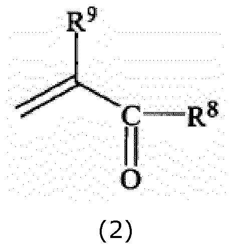 wobei R⁸ unabhängig aus der Gruppe ausgewählt ist, die aus Folgenden besteht:
(i) OH;
(ii) linearem oder verzweigtem C₁-C₁₂-Alkoxy, das gegebenenfalls mit 1 bis 6 Hydroxygruppen substituiert ist;
(iii) Amid gemäß der Formel -N(R¹⁰)₂, wobei R¹⁰ Wasserstoff oder lineares oder verzweigtes C₁-C₆-Alkyl, das gegebenenfalls mit 1 bis 6 Hydroxygruppen substituiert ist, sein kann;
(iv) Ammoniumsalz gemäß der Formel -[N(R¹⁰)₃]⁺X⁻, wobei R¹⁰ wie für (iii) definiert ist und X ein Halogenatom ist; und
(v) einer Gruppe gemäß Formel (3) 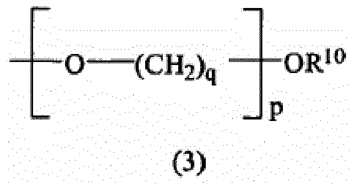 wobei R¹⁰ wie für (iii) definiert ist und p = 1 bis 50 ist und q = 2 oder 3 ist; und
wobei R⁹ Wasserstoff oder Methyl ist;
(b) linearem oder verzweigtem C₁-C₁₂-Alkylvinylether;
(c) Vinylalkohol;
(d) C₂-C₁₂-α-Alkenylen-ω-sulfonat, das ein Erdalkalimetallkation oder ein Alkalimetallkation aufweist;
(e) C₇-C₁₂-Vinylarylsulfonat gemäß Formel (4) 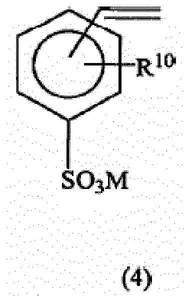 wobei R¹⁰ wie für (iii) definiert ist und M ein Erdalkalimetall- oder Alkalimetallkation ist;
(f) CH₂=CH-R¹¹, wobei R¹¹ aus der Gruppe ausgewählt ist, die aus Pyrrolyl, Imidazolyl, Pyrazolyl, Pyridinyl, Pyrazinyl, Pyrimidinyl, Pyrrolidyl, Indolyl, Indolizinyl, Indazolyl, Purinyl, Chinolizinyl, Chinolinyl, Isochinolinyl, Phthalizinyl, Naphthypyridinyl, Chinoxalinyl, Chinazolinyl, Cinnolinyl, Pteridinyl, Pyrrolidonyl, Pyrrolinyl, Pyrrolidinyl, Imidazolidinyl, Imidazolinyl, Pyrazolidinyl, Pyrazolinyl, Piperidyl, Piperazinyl, Indolinyl, Isoindolinyl, Morpholinyl, Isoxazolyl, Furazinyl und Isothiazolyl besteht;
(g) CH₂=CH-O-C(O)R¹², wobei R¹² aus der Gruppe ausgewählt ist, die aus linearem oder verzweigtem C₁-C₆-Alkyl besteht;
(h) CH₂=CH-CH₂OR¹³, wobei R¹³ aus der Gruppe ausgewählt ist, die aus Wasserstoff und linearem oder verzweigtem C₁-C₆-Alkyl besteht; und
(i) N-Vinyllactamen gemäß Formel (5) 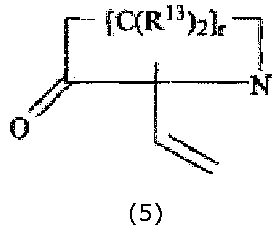
wobei R¹³ wie für (h) definiert ist und r = 2 bis 6 beträgt.

13. Hydrogel gemäß einem der vorstehenden Ansprüche, wobei das Hydrogel eine biologisch aktive oder pharmazeutisch aktive Verbindung oder eine bioaktive Spezies umfasst.

14. Verwendung des Hydrogels gemäß einem der Ansprüche 1 bis 12 in pharmazeutischen oder biomedizinischen Zusammensetzungen.

15. Verfahren zur Herstellung eines Hydrogels gemäß einem der Ansprüche 1 bis 12, wobei:
(a) das Hydrogel bei Temperaturen zwischen 40 °C und 95 °C in einem wässrigen Lösungsmittel aufgelöst wird und anschließend auf Temperaturen zwischen 0 °C und 40 °C abgekühlt wird;
(b) das Hydrogel in einem wässrigen Lösungsmittel bei einer Temperatur zwischen 0 °C und 60 °C aufquellen gelassen wird; oder
(c) das Hydrogel in einem organischen wassermischbaren Lösungsmittel oder Lösungsmittelgemisch aufgelöst oder dispergiert wird, gefolgt von der Zugabe von Wasser und anschließend der optionalen Entfernung des organischen Lösungsmittels durch Verdampfen im Vakuum oder Entfernung des organischen Lösungsmittels durch Waschen mit Wasser.

## Revendications

1. Hydrogel comprenant :
(a) de 0,3 à 50,0 % en poids, par rapport au poids total de l'hydrogel, d'un gélifiant aqueux, le gélifiant aqueux pouvant être obtenu par conversion d'une charpente hydrophile P, où P est choisi dans le groupe constitué par les polyéthers, les polyesters et les copolymères de ces polymères, dans une première étape, en un prépolymère à fonctionnalité amine de formule P-[L]ₙ, dans une deuxième étape, un motif de liaison hydrogène simple est introduit aux extrémités du lieur hydrophobe L par réaction du prépolymère à fonctionnalité amine P-[L]ₙ avec un composé mono- ou di-fonctionnel choisi dans le groupe constitué par les (di)isocyanates, les (di)thioisocyanates, les (di)amines éventuellement activées, les chlorures de (di)acides, les (di)esters éventuellement activés, les (di)ols éventuellement activés, les (di)acrylamides, les (di)méthacrylamides et les (di)thiols éventuellement activés ; où P et L sont connectés l'un à l'autre par des fragments choisis dans le groupe constitué par les fragments (thio)urée, (thio)uréthane, amide, ester, carbonate, amine secondaire, amine tertiaire et éther,
où :
n est situé dans la plage allant de 1,8 à 10 ;
L est un lieur hydrophobe choisi dans le groupe constitué par les groupes alkylène en C₂ à C₂₄, les groupes arylène en C₆ à C₂₄, les groupe alcarylène en C₇ à C₂₄ et les groupes arylalkylène en C₇ à C₂₄, cycliques, linéaires ou ramifiés, les groupes alkylène, groupes arylène, groupes alcarylène et groupes arylalkylène comprenant éventuellement, mais de façon non préférable, de 1 à 5 hétéroatomes choisis dans l'ensemble constitué par O, N et S ; et
(b) de 50,0 à 99,7 % en poids d'eau.

2. Hydrogel selon la revendication 1, dans lequel le composé mono- ou di-fonctionnel est choisi dans le groupe constitué par les (di)isocyanates, les (di)amines activées et les (di)esters activés.

3. Hydrogel selon la revendication 1 ou la revendication 2, dans lequel le composé mono- ou di-fonctionnel est choisi dans le groupe constitué par les (di)isocyanates.

4. Hydrogel selon l'une quelconque des revendications 1 à 3, dans lequel le motif de liaison hydrogène simple est choisi dans le groupe constitué par thio(urée), amide et (thio)uréthane.

5. Hydrogel selon l'une quelconque des revendications 1 à 4, lequel hydrogel comprend un gélifiant aqueux qui peut être obtenu sans utilisation d'un catalyseur à base de métal toxique.

6. Hydrogel selon l'une quelconque des revendications 1 à 4, dans lequel le lieur L est une diamine et dans lequel la charpente de polymère hydrophile P est de formule P-(OH)ₙ dans laquelle n est situé dans la plage allant de 1,8 à 10.

7. Hydrogel selon l'une quelconque des revendications 1 à 5, dans lequel le gélifiant aqueux a une masse moléculaire de 1 200 à 1 000 000.

8. Hydrogel selon l'une quelconque des revendications 1 à 6, dans lequel la charpente de polymère hydrophile P a une masse moléculaire de 250 à 200 000.

9. Hydrogel selon l'une quelconque des revendications 1 à 7, dans lequel la charpente de polymère hydrophile P est téléchélique.

10. Hydrogel selon l'une quelconque des revendications 1 à 8, dans lequel la charpente de polymère hydrophile P est de formule P-(OH)₂.

11. Hydrogel selon la revendication 9 ou la revendication 10, dans lequel la charpente de polymère hydrophile P-(OH)₂ est un polyéthylèneglycol.

12. Hydrogel selon l'une quelconque des revendications 1 à 8, dans lequel la charpente de polymère hydrophile greffé fonctionnel P-(OH)ₙ est préparée à partir de polymères pouvant être obtenus par (co)polymérisation de monomères vinyliques, les monomères vinyliques étant choisis dans le groupe constitué par :
(a) les monomères de formule (2) 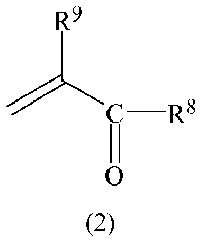 dans laquelle R⁸ est indépendamment choisi dans l'ensemble constitué par :
(i) OH ;
(ii) alcoxy en C₁ à C₁₂ linéaire ou ramifié, éventuellement substitué par 1 à 6 groupes hydroxy ;
(iii) amide de formule -N(R¹⁰)₂ dans laquelle R¹⁰ peut être l'hydrogène ou un alkyle en C₁ à C₆ linéaire ou ramifié, éventuellement substitué par 1 à 6 groupes hydroxy ;
(iv) un sel d'ammonium de formule -[N(R¹⁰)₃]⁺X⁻ dans laquelle R¹⁰ est tel que défini en (iii) et X est un atome d'halogène ; et
(v) un groupe de formule (3) 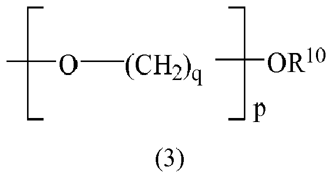
dans laquelle R¹⁰ est tel que défini en (iii) et p vaut de 1 à 50 et q vaut 2 ou 3 ; et
dans laquelle R⁹ est hydrogène ou méthyle ;
(b) un alkylvinyléther linéaire ou ramifié en C₁ à C₁₂ ;
(c) l'alcool vinylique ;
(d) un α-alcénylène-ω-sulfonate en C₂ à C₁₂ ayant un cation de métal alcalino-terreux ou un cation de métal alcalin ;
(e) un vinylarylsulfonate en C₇ à C₁₂ de formule (4) 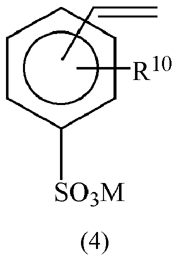 dans laquelle R¹⁰ est tel que défini en (iii) et M est un métal alcalino-terreux ou un cation de métal alcalin ;
(f) CH₂=CH-R¹¹, où R¹¹ est choisi dans le groupe constitué par pyrrolyle, imidazolyle, pyrazolyle, pyridinyle, pyrazinyle, pyrimidinyle, pyrrolidyle, indolyle, indolizinyle, indazolyle, purinyle, quinolizinyle, quinolinyle, isoquinolinyle, phtalizinyle, naphtypyridinyle, quinoxalinyle, quinazolinyle, cinnolinyle, ptéridinyle, pyrrolidonyle, pyrrolinyle, pyrrolidinyle, imidazolidinyle, imidazolinyle, pyrazolidinyle, pyrazolinyle, pipéridyle, pipérazinyle, indolinyle, isoindolinyle, morpholinyle, isoxazolyle, furazinyle et isothiazolyle;
(g) CH₂=CH-O-C(O)R¹², où R¹² est choisi dans l'ensemble constitué par les radicaux alkyle en C₁ à C₆ linéaires ou ramifiés ;
(h) CH₂=CH-CH₂OR¹³, où R¹³ est choisi dans l'ensemble constitué par l'hydrogène et les radicaux alkyle en C₁ à C₆ linéaires ou ramifiés ; et
(i) les N-vinyl-lactames de formule (5) 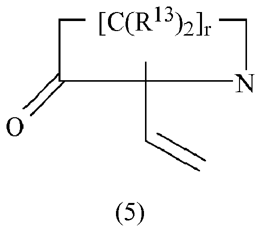 dans laquelle R¹³ est tel que défini en (h) et r vaut de 2 à 6.

13. Hydrogel selon l'une quelconque des revendications précédentes, lequel hydrogel comprend un composé biologiquement actif ou pharmaceutiquement actif ou une espèce bioactive.

14. Utilisation de l'hydrogel selon l'une quelconque des revendications 1 à 12 dans des compositions pharmaceutiques ou biomédicales.

15. Procédé pour préparer un hydrogel selon l'une quelconque des revendications 1 à 12, dans lequel :
(a) l'hydrogel est dissous dans un solvant aqueux à une température comprise entre 40 °C et 95 °C, opération suivie d'un refroidissement à une température comprise entre 0 °C et 40 °C ;
(b) l'hydrogel est gonflé dans un solvant aqueux à une température comprise entre 0 °C et 60 °C ; ou
(c) l'hydrogel est dissous ou dispersé dans un solvant organique miscible à l'eau ou un mélange de tels solvants, opération suivie de l'addition d'eau et ensuite de l'élimination éventuelle du solvant organique avec évaporation sous vide ou de l'élimination du solvant organique par lavage à l'eau.
